# EUROPÄISCHE PATENTSCHRIFT

(11) **EP 1 978 970 B1**
(45) Veröffentlichungstag und Bekanntmachung des Hinweises auf die Patenterteilung: **09.03.2011**
(21) Anmeldenummer: 07722763.5
(22) Anmeldetag: 23.01.2007
(51) Int. Cl.: A61K 31/565, A61K 31/57, A61P 15/18

(54) **KONTRAZEPTIVUM**
CONTRACEPTIVE
AGENT CONTRACEPTIF

(30) Priorität: 24.01.2006 DE 102006003509
(43) Veröffentlichungstag der Anmeldung: 15.10.2008
(73) Patentinhaber: Grünenthal GmbH, 52078 Aachen (DE)
(72) Erfinder: SCHRAMM, Georg, 52224 Stolberg (DE); KNEIP, Christa, 52080 Aachen (DE); SCHNEIDER, Johannes, 52223 Stolberg (DE)
(74) Vertreter: Kutzenberger, Helga
(86) Internationale Anmeldenummer: PCT/EP2007/000551
(87) Internationale Veröffentlichungsnummer: WO 2007/098828

(56) Entgegenhaltungen:
- DE-A1- 4 321 957
- DE-A1- 4 339 934
- BUNDESVERBAND DER PHARMAZEUTISCHEN INDUSTRIE E.V UND WEITERE: "Rote Liste 2002" 2002, ROTE LISTE SERVICE GMBH, FRANKFURT/MAIN , GERMANY , XP002432757 siehe "Belara" N.: 76131
- HONMA S ET AL: "IDENTIFICATION AND ANTI-ANDROGENIC ACTIVITY OF THE METABOLITES OF 17ALPHA-ACETOXY-6-CHLOROPREGNA-4,6-DIENE-3 ,20-DIONE (CHLORMADINONE ACETATE) IN THE RAT, RABBIT, DOG AN DMAN" CHEMICAL AND PHARMACEUTICAL BULLETIN, PHARMACEUTICAL SOCIETY OF JAPAN, TOKYO, JP, Bd. 25, Nr. 8, 25. August 1977 (1977-08-25), Seiten 2019-2031, XP009083454 ISSN: 0009-2363

## Beschreibung

Die vorliegende Erfindung betrifft eine Darreichungsform zur hormonalen Kontrazeption bestehend aus einer bestimmten Anzahl von hormonhaltigen Tages-Einheiten, die eine Hormonkombination bestehend aus wenigstens einem Östrogen auswählt aus der Gruppe bestehend aus Ethinylestradiol (I) und Östradiol (II) als Östrogenkomponente und aus wenigstens einem Metaboliten des Chlormadinonacetats ausgewählt aus der Gruppe umfassend 3α-Hydroxy-6-chlor-17α-acetoxy-4,6-pregnadien-20-on (3α-Hydroxy-chlormadinonacetat), 3β-Hydroxy-6-chlor-17α-acetoxy-4,6-pregnadien-20-on (3β-Hydroxy-chlormadinonacetat) gegebenenfalls gemischt mit Chlormadinonacetat und/oder 3α-Hydroxy-17α-acetoxy-5β-pregnan-20-on und/oder 3β-Hydroxy-17α-acetoxy-5β-pregnan-20-on als Gestagenkomponente aufweist zur ununterbrochenen, täglichen, oralen Verabreichung der hormonhaltigen Tages-Einheiten, gegebenenfalls in Kombination mit 7 bis 3 hormonfreien Tages-Einheiten.

Von hormonalen Kontrazeptiva enthaltend eine Hormonkombination ist bekannt, dass Chlormadinonacetat eine wirksame Gestagenkomponente darstellt. Es ist auch bekannt, dass die Metabolisierung des Chlormadinonacetats u. a. über die Metaboliten 3α-Hydroxy-6-chlor-17α-acetoxy-4,6-pregnandien-20-on (3α-Hydroxychlormadinonacetat), 3β-Hydroxy-6-chlor-17α-acetoxy-4,6-pregnandien-20-on (3β-Hydroxy-chlormadinonacetat), 3α-Hydroxy-17α-acetoxy-5β-pregnan-20-on bzw. 3β-Hydroxy-17α-acetoxy-5β-pregnan-20-on erfolgt.

DE 43 39 934, DE 43 21 957und BUNDESVERBAND DER PHARMAZEUTISCHEN INDUSTRIE E.V UND WEITERE: "Rote Liste 2002" 2002, ROTE LISTE SERVICE GMBH, FRANKFURT/MAIN , GERMANY, beschreiben Präparate enthaltend Ethinylestradiol und Chlormadinonacetat. Belara^{R} ist ein kommerziell erhältliches Kontrazeptivum enthaltend Ethinylestradiol und Chlormadinonacetat.

Es wurde nun überraschenderweise gefunden, dass sich die Metaboliten 3α-Hydroxy-chlormadinonacetat und/oder 3β-Hydroxy-chlormadinonacetat gegebenenfalls in Mischung mit Chlormadinonacetat und/oder 3α-Hydroxy-17α-acetoxy-5β-pregnan-20-on und/oder 3β-Hydroxy-17α-acetoxy-5β-pregnan-20-on hervorragend als Gestagenkomponente in Kombination mit einer Östrogenkomponente zur Kontrazeption eignen.

Weiterhin wurde gefunden, dass die genannten Gestagenkomponenten Stimmungsschwankungen bei Frauen positiv beeinflussen können.

Da nämlich viele Frauen während ihres Menstruationszyklus ohne erkennbare äußere Einflüsse oder Störungen oder ohne Beschwerden oder Störungen verbunden mit dem Menstruationszyklus unter Stimmungsschwankungen leiden, besteht ein Bedarf, diese Stimmungsschwankungen, die von Frauen als psychische Beeinträchtigung empfunden werden und deren Lebensqualität beeinträchtigen, zumindest zu lindern und damit die Stimmung während des gesamten Ablaufs eines Menstruationszyklus so zu verbessern, dass sogar eine Stimmungsaufhellung insgesamt erfolgt.

Aus verständlichen Gründen möchten Frauen, die eine wirksame Kontrazeption anstreben und unter solchen Stimmungsschwankungen leiden, nicht mehrere Präparate einnehmen müssen, sondern nur eine, vorzugsweise 1 x-tägliche Einnahme vornehmen zu müssen. Diese Aufgabe wird auch durch das erfindungsgemäße Kontrazeptivum gelöst.

Das erfindungsgemäße Kontrazeptivum besteht aus einer bestimmten Anzahl von hormonhaltigen Tages-Einheiten, die eine Hormonkombination bestehend aus wenigstens einem Östrogen auswählt aus der Gruppe bestehend aus Ethinylestradiol (I) und Östradiol (II) als Östrogenkomponente und aus wenigstens einem Metaboliten des Chlormadinonacetats ausgewählt aus der Gruppe umfassend 3α-Hydroxy-6-chlor-17α-acetoxy-4,6-pregnadien-20-on (3α-Hydroxy-chlormadinonacetat), 3β-Hydroxy-6-chlor-17α-acetoxy-4,6-pregnadien-20-on (3β-Hydroxychlormadinonacetat) gegebenenfalls gemischt mit Chlormadinonacetat und/oder 3α-Hydroxy -17α-acetoxy-5β-pregnan-20-on und/oder 3β-Hydroxy-17α-acetoxy-5β-pregnan-20-on als Gestagenkomponente aufweist, zur ununterbrochenen, täglichen, oralen Verabreichung der hormonhaltigen Tages-Einheiten, gegebenenfalls in Kombination mit 7 bis 3 hormonfreien Tages-Einheiten.

Die erfindungsgemäß zum Einsatz kommende Gestagenkomponente ist eine der folgenden Komponenten a) bis f)
a) 3α-Hydroxy-chlormadinonacetat oder
b) 3β-Hydroxy-chlormadinonacetat oder
c) eine Mischung von a) und b) in einem beliebigen Mischungsverhältnis oder
d) eine Mischung von a) und/oder b) mit bis zu 20 Gew.%, bezogen auf die Gesamtmischung von 3α-Hydroxy-17α-acetoxy-5β-pregnan-20-on und/oder 3β Hydroxy-17α-acetoxy-5β-pregnan-20-on, wobei die letztgenannten Metaboliten in einem beliebigen Mischungsverhältnis vorliegen können, oder
e) eine Mischung von Chlormadinonacetat mit a), b) oder c) in Mischungsverhältnis von 10 bis 90 Gew.% Chlormadinonacetat und 90 bis 10 Gew.% von a) und/oder b), bezogen auf die Gesamtmischung, oder
f) eine Mischung von Chlormadinonacetat mit c) in einem Mischungsverhältnis von 10 bis 90 Gew.% Chlormadinonacetat und 90 bis 10 Gew.% von c), bezogen auf die Gesamtmischung und bis zu 20 Gew.%, bezogen auf die Gesamtmischung, 3α-Hydroxy-17α-acetoxy-5β-pregnan-20-on und/oder 3β-Hydroxy-17α-acetoxy-5β-pregnan-20-on, wobei die Gesamtmischung immer 100 Gew.% ergeben muss.

Eine erfindungsgemäße Tages-Einheit enthält vorzugsweise eine Hormonkombination bestehend aus 5 bis 50 µg Ethinylestradiol und/oder 0,5 bis 4 mg Östradiol und 1 bis 10 mg einer der genannten Gestagenkomponenten a) bis f) und gegebenenfalls üblichen Hilfsstoffen.

Weiterhin bevorzugt ist in einer Tages-Einheit eine Hormonkombination bestehend aus mindestens 15 µg Ethinylestradiol und/oder mindestens 0,5 mg Östradiol und mindestens 1 mg der genannten Gestagenkomponenten a) bis f).

Ganz besonders bevorzugt sind Tages-Einheiten, die jeweils aus einer Hormonkombination bestehend aus jeweils mindestens15 µg, vorzugsweise 20 µg oder 30 µg Ethinylestradiol und/oder mindestens 0,5 mg, vorzugsweise 1 mg oder 2 mg Östradiol, und jeweils mindestens 1 mg, vorzugsweise 2, 3, 4 oder 5 mg einer der Gestagenkomponenten a) bis f) und gegebenenfalls aus üblichen Hilfsstoffen bestehen.

Aufgrund der ausgezeichneten kontrazeptionellen Wirkung eignet sich auch ganz besonders bevorzugt eine Hormonkombination aus jeweils 20 µg Ethinylestradiol und/oder 1 mg Östradiol und ≥2 mg der Gestagenkomponenten a) bis f) zur Herstellung einer Tages-Einheit der erfindungsgemäßen Darreichungsform.

Das erfindungsgemäße Kontrazeptivum wird vorzugsweise in Form von Tabletten formuliert, die neben der aufgeführten Hormonkombination gegebenenfalls noch übliche Hilfsstoffe enthalten.

Diese Tabletten werden insbesondere in Form von mindestens 21, vorzugsweise 21 bis 25, die Hormonkombination enthaltenden Tages-Einheiten, die für eine ununterbrochene, orale Einnahme gedacht sind, gefolgt von einer 3 bis 7-tägigen Einnahmepause oder in Kombination mit 7 bis 3 hormonfreien Tages-Einheiten für eine ununterbrochene, orale Einnahme durch Frauen bereitgestellt.

Zur Vermeidung von möglichst vielen Blutungen, wie Zwischen- oder Entzugsblutungen, kann das erfindungsgemäße Kontrazeptivum auch in Form von die Hormonkombination enthaltenden Tages-Einheiten für eine ununterbrochene Verabreichung über mehrere Jahre, vorzugsweise bis zu 2 Jahren, besonders bevorzugt bis zu 1 Jahr, gegebenenfalls in Kombination mit 7 bis 3 hormonfreien Tages-Einheiten zur ununterbrochenen Verabreichung oder unmittelbar gefolgt von einer 7 bis 3-tägigen Einnahmepause zur Verfügung gestellt werden.

Das erfindungsgemäße Kontrazeptivum kann aber auch in einer Darreichungsform mit weniger als 365 die Hormonkombination enthaltenden Tages-Einheiten, wie z. B. mit 77 bis 193 bzw. 42 bis 52, die Hormonkombination enthaltenden Tages-Einheiten zur ununterbrochenen oralen Verabreichung, gefolgt von einer Einnahmepause über 7 bis 3 Tage oder in Kombination mit 7 bis 3 hormonfreien Tages-Einheiten zur ununterbrochenen Verabreichung bereitgestellt werden.

Wie bereits dargelegt, kann anstelle der 7 bis 3 hormonfreien Tages-Einheiten auch eine entsprechend lange Einnahmepause gemacht werden. Dementsprechend kann die orale Darreichungsform mit den vorstehend aufgeführten Zahl an die Hormonkombination enthaltenden Tages-Einheiten auch als Kit vorliegen, das mehrere dieser Darreichungsformen zur fortgeführten Einnahme, unterbrochen von einer entsprechenden Einnahmepause umfasst. Selbstverständlich kann ein Kit auch mehrere orale Darreichungsformen umfassen, die eine ununterbrochene Einnahme von die Hormonkombination enthaltenden Tages-Einheiten in Kombination für eine ununterbrochene Einnahme der aufgeführten Zahl von hormonfreien Tages-Einheiten aufweist.

Vorzugsweise weist jede der die Hormonkombination enthaltenden Tages-Einheiten dieselbe Menge der Östrogenkomponente bzw. der Gestagenkomponente auf, d. h. sowohl die Menge an Ethinylestradiol und/oder Östradiol als auch an einer der Gestagenkomponenten a) bis f) wird über einen Einnahmezyklus, der wie vorstehend ausgeführt bis zu mehreren Jahren dauern kann, konstant gehalten.

In einer weiteren Ausführuhgsform können die die Hormonkombination enthaltenden Tages-Einheiten gemäß einem zweiphasigen oder dreiphasigen Einnahmezyklus über 21 bis 25 Tage in ihrem Gehalt an Ethinylestradiol oder Östradiol bzw. der Gestagenkomponenten a) bis f) in bekannter Weise variieren, um eine kontrazeptive Wirkung zu erzielen.

Dazu weisen die die Hormonkombination enthaltenden Tages-Einheiten vorzugsweise eine Menge von gleich oder weniger 20 µg Ethinylestradiol, vorzugsweise 20 µg oder 30 µg Ethinylestradiol oder 1 mg Östradiol über alle Phasen und eine phasenabhängige, unterschiedliche Menge von 2 bis 5 mg einer der Gestagenkomponenten a) bis f) auf.

Bei einem Zweiphasen-Kontrazeptivum wird der Einnahmezyklus vorzugsweise mit einer täglichen Einnahme einer Tages-Einheit enthaltend 2 bis 3 mg einer der Gestagenkomponenten a) bis f) neben Ethinylestradiol oder Östradiol als Gestagenkomponente über einen Zeitraum von 7 bis 12 Tagen, gefolgt von einer täglichen Einnahme einer Tages-Einheit von 3 bis 4 mg derselben Gestagenkomponente über einen Zeitraum von 9 bis 18 Tagen begonnen, wobei die Menge der Gestagenkomponente in der ersten Phase immer geringer als in der zweiten Phase ist, aber jeweils konstant bleibt und die Menge an Ethinylestradiol bzw. Östradiol pro Tages-Einheit über beide Phasen konstant unverändert bei jeweils 20 µg oder 30 µg bzw. 1 mg liegt.

Sofern das erfindungsgemäße Kontrazeptivum als Dreiphasen-Kontrazeptivum eingenommen wird, beginnt der Einnahmezyklus vorzugsweise mit einer ununterbrochenen, täglichen Einnahme einer Tages-Einheit enthaltend 2 bis 3 mg einer der Gestagenkomponenten a) bis f) neben Ethinylestradiol oder Östradiol als Östrogenkomponente über einen Zeitraum von 6 bis 7 Tagen, gefolgt von einer täglichen Einnahme einer Tages-Einheit enthaltend 3 mg einer der genannten Gestagenkomponenten neben Ethinylestradiol oder Östradiol über einen ununterbrochenen Zeitraum von 5 bis 9 Tagen und endet mit einer täglichen, ununterbrochenen Einnahme einer Tages-Einheit enthaltend 3 bis 5 mg einer der genannten Gestagenkomponenten über einen Zeitraum von 5 bis 14 Tagen. Auch bei einem Dreiphasen-Kontrazeptivum enthalten die Tages-Einheiten von Phase zu Phase jeweils unterschiedliche Mengen der identischen Gestagenkomponenten, wobei von der ersten bis zur dritten Phase die Menge der Gestagenkomponenten pro Tages-Einheit ansteigt, innerhalb einer Phase aber konstant bleibt, ebenso wie die Menge an Ethinylestradiol oder Östradiol über alle Phasen in einer identischen Menge pro Tages-Einheit konstant bleibt.

Sowohl bei einem Arzneimittel, das eine sogenannte Zweiphasen-Kontrazeptionswirkung entfaltet als auch bei einer Dreiphasen-Kontrazeptionswirkung enthalten die hormonhaltigen Tages-Einheiten vorzugsweise jeweils 20 bis 50 µg, besonders bevorzugt jeweils 30 µg, ganz besonders bevorzugt jeweils 20 µg Ethinylestradiol und/oder vorzugsweise jeweils 1 bis 4 mg, bevorzugt 2 mg, besonders bevorzugt 1 mg Östradiol als Östrogenkomponente. Außerdem ist zur Erzielung einer maximalen Sicherheit in der kontrazeptiven Wirkung die Einhaltung einer ununterbrochen aufeinanderfolgenden Einnahme von 21 bis 25 die Hormonkombination enthaltenden Tages-Einheiten pro Einnahmezyklus besonders wichtig. Nach der Einnahme der die Hormonkombination enthaltenden Tages-Einheiten kann sich eine ununterbrochene Einnahme über 7 bis 3 Tage von hormonfreien Tages-Einheiten oder eine dementsprechend gleich lange Einnahmepause unmittelbar anschließen.

Durch die Einnahme des erfindungsgemäßen Kontrazeptivums gelingt es nicht nur, eine ausgezeichnete kontrazeptionelle Wirkung zu erzielen, sondern überraschenderweise auch bei Frauen, die unter menstruationszyklusabhängigen Stimmungsschwankungen leiden, diese nicht nur zu lindern, sondern sogar zu verhindern, wobei nicht nur eine Verschlechterung des Gemütszustandes bis hin zu einem seelischen Tiefstand bei solchen Frauen verhindert, sondern auch das seelische Befinden, d. h. den Gemütszustand einer Frau während ihres gesamten Menstruationszyklus so verbessert wird, dass insgesamt eine Stimmungsaufhellung dabei auftritt. Insbesondere bei einer monophasigen Einnahme ist diese Wirkung zu erzielen.

Das erfindungsgemäße Kontrazeptivum liegt vorzugsweise als orale Darreichungsform, ganz besonders bevorzugt in Form von Tabletten, vor. Dabei entspricht eine Tages-Einheit jeweils einer Tablette. Die Tabletten werden entsprechend einen Einnahmezyklus, vorzugsweise in Blistern verpackt, vorzugsweise unter Kennzeichnung der jeweils einzunehmenden Tages-Einheit, und als Packung enthaltend mindestens einen solchen Blister, vorzugsweise mindestens 3 Blister für die jeweilige Anzahl von Einnahmezyklen bzw. für eine angestrebte ununterbrochene Verabreichung marktgeführt werden.

### In vitro-Daten

### 1) Bestimmung der Affinität von 3-α-OH-CMA bzw. 3-β-OH-CMA zum humanen Progesteronrezeptor

Die nachfolgende Bestimmung der Affinität von 3-α-OH-CMA bzw. 3-β-OH-CMA zum humanen Progesteronrezeptor basiert auf der Veröffentlichung der experimentellen Vorschriften von Eckert et al. (Cancer Research, 1982, V42, p.139-144). Die entsprechende Offenbarung wird hiermit als Referenz eingeführt und ist Teil der Offenbarung der vorliegenden Anmeldung.

Die cytosolischen Fraktionen von MCF-7-Zellen, welche den humanen Progesteronrezeptor enthalten, werden verwendet. Als Referenzsubstanz wird 2 nM [³H] R 5020, zur Bestimmung der unspezifischen Bindung wird 1 µM R 5020 eingesetzt. Die Inkubationsdauer der Substanzen ([³H] R 5020, 3-α-OH-CMA bzw. 3-β-OH-CMA) am Rezeptor beträgt jeweils 20 Stunden bei einer Temperatur von ca. 4°Celsius. Stammlösungen (5 x 10⁻² M) von 3-α-OH-CMA bzw. 3-β-OH-CMA werden jeweils in 75%-igem DMSO 1:10 vorverdünnt und anschließend 1:5 in 25%-igem DMSO weiterverdünnt. Die finalen Konzentrationen von 3-α-OH-CMA bzw. 3-β-OH-CMA im Test sind 3x10⁻¹⁰ M, 3x10⁻⁹ M, 1x10⁻⁸ M, 3x10⁻⁸ M, 1x10⁻⁷ M, 3x10⁻⁷ M, 1 x10⁻⁶ M und 1x10⁻⁵ M. Nach der o.g. Inkubationsdauer der Substanzen werden die Inkubationsansätze nach Standardvorschriften filtriert, gewaschen und die Radioaktivität der Filter mit einem Scintillationsmeßgerät bestimmt. Derartige Standardvorschriften sind dem Fachmann bekannt. Die Experimente werden jeweils in Doppelansätzen durchgeführt.

Die entsprechenden IC₅₀-Werte werden über eine nicht-lineare Regressionsanalyse der Verdrängungskurven über die Hill-Kurvenangleichungsformel berechnet. Ein derartiges Berechnungsverfahren ist dem Fachmann bekannt.

Die entsprechenden Kᵢ-Werte (Inhibitionskonstante) werden über die Cheng-Prusoff-Gleichung bestimmt (Kᵢ = IC₅₀/(1+(L/K_{D})), wobei L der Konzentration des Radioliganden im Test und K_{D} der Affinität des Radioliganden zum Rezeptor entspricht).

Die Messwerte sind in der nachfolgenden Tabelle zusammengefasst.

### 2) Bestimmung der Affinität von 3-α-OH-CMA bzw. 3-β-OH-CMA zum humanen Androgenrezeptor

Die nachfolgende Bestimmung der Affinität von 3-α-OH-CMA bzw. 3-β-OH-CMA zum humanen Androgenrezeptor basiert auf der Veröffentlichung der experimentellen Vorschriften von Zava et al. (Endocrinology, 1979, V104, p.1007-1012). Die entsprechende Offenbarung wird hiermit als Referenz eingeführt und ist Teil der Offenbarung der vorliegenden Anmeldung.

Die cytosolischen Fraktionen von LNCaP-Zellen, welche den humanen Androgenrezeptor enthalten, werden verwendet. Als Referenzsubstanz wird 0.5 nM [³H] Methyltrienolon, zur Bestimmung der unspezifischen Bindung wird 1 µM Miboleron eingesetzt. Die Inkubationsdauer der Substanzen ([³H] Methyltrienolon, 3-α-OH-CMA bzw. 3-β-OH-CMA) am Rezeptor beträgt jeweils 24 Stunden bei einer Temperatur von ca. 4°Celsius. Die Stammlösungen, die Verdünnungsreihen von 3-α-OH-CMA bzw. 3-β-OH-CMA, die Waschschritte, die Bestimmung der Radioaktivität, sowie die Methoden zur Berechnungen der jeweiligen IC₅₀ und Kᵢ-Werte entsprechen dem unter Punkt 1) beschriebenen experimentellen Protokoll.

Die Messwerte sind in der nachfolgenden Tabelle zusammengefasst.

| **Progesteronrezeptor (human)** | | |
|---|---|---|
| **Substanz** | IC₅₀ [nM] | Kᵢ [nM] |
| 3-α-OH-CMA | 39 | 13 |
| 3-β-OH-CMA | 18 | 6 |

| **Androgenrezeptor (human)** | | |
|---|---|---|
| **Substanz** | IC₅₀ [nM] | Kᵢ [nM] |
| 3-α-OH-CMA | 100 | 83 |
| 3-β-OH-CMA | 25 | 20 |

Aus den Daten geht hervor, dass sowohl 3-α-OH-CMA als auch 3-β-OH-CMA eine hohe Affinität zum humanen Progesteronrezeptor und zum humanen Androgenrezeptor haben. Daraus ist deren jeweilige kontrazeptive und anti-androgene Wirkung ableitbar.

Es ist bekannt, dass das endogen gebildete Neurosteroid Allopregnanolon einen positiven Effekt auf die Stimmung hat. Plasmakonzentrationen von Allopregnanolon sind in Patientinnen mit depressiver Stimmung vermindert. Die positive Wirkung des Allopregnanolons auf die Stimmung wird auf dessen Wechselwirkung mit GABA_{A}-Rezeptoren zurückgeführt. An diesem Rezeptor des Zentralnervensystems wirkt Allopregnanolon als positiver allosterischer Modulator und führt damit zu anxiolytischen und stimmungsaufhellenden Effekten.

Es wurde gefunden, dass 3-α-OH-CMA bzw. 3-β-OH-CMA Bindungseigenschaften an GABA_{A}-Rezeptoren zeigen, die denen des Allopregnanolons sehr ähnlich sind. Allopregnanolon beeinflusst die Bindung von radioaktiv markiertem Muscimol (Agonist) an diese Rezeptoren.

### 3) Einfluss von Allopregnanolon, 3-α-OH-CMA bzw. 3-β-OH-CMA auf die Bindung von Muscimol an GABA_{A}-Rezeptoren (Rattenhirn)

Die nachfolgenden Bestimmungen des Einflusses von Allopregnanolon, 3-α-OH-CMA bzw. 3-β-OH-CMA auf die Bindung von Muscimol an GABA_{A}-Rezeptoren (Ratte) basieren auf der Veröffentlichung der experimentellen Vorschriften von Snodgrass, S.R. (Nature, 1979, V273, p.392-394). Die entsprechende Offenbarung wird hiermit als Referenz eingeführt und ist Teil der Offenbarung der vorliegenden Anmeldung.

### A) Allopregnanolon

Membranpräparationen aus cerebralem Rattencortexmaterial werden hergestellt. Als Referenzsubstanz wird 5 nM [³H] Muscimol, zur Bestimmung der unspezifischen Bindung wird 10 µM Muscimol eingesetzt. Die Inkubationsdauer der Substanzen ([³H] Muscimol, Allopregnanolon) am Rezeptor beträgt jeweils 10 Minuten bei einer Temperatur von ca. 4°Celsius. Die Stammlösung (5 x 10⁻² M) von Allopregnanolon wird jeweils in 75%-igem DMSO 1:10 vorverdünnt und anschließend 1:5 in 25%-igem DMSO weiterverdünnt. Die finale Konzentration von Allopregnanolon im Test ist jeweils 1x10⁻¹⁰ M, 1x10⁻⁹ M, 1x10⁻⁸ M, 1x10⁻⁷ M, 1x10⁻⁶M und 1x10⁻⁵ M. Die Waschschritte, sowie die Bestimmungen der Radioaktivität werden wie vorstehend unter Punkt 1 beschrieben durchgeführt.

Es kann gezeigt werden, dass bei einer Konzentration von 1 µM Allopregnanolon die Bindung des radioaktiv markierten Muscimols (5 nM) um 38% erhöht wird.

### B) 3-α-OH-CMA bzw. 3-β-OH-CMA

Die Bestimmung des Einflusses von 3-α-OH-CMA bzw. 3-β-OH-CMA auf die Bindung von Muscimol an GABA_{A}-Rezeptoren (Ratte) erfolgt analog zu der vorstehend beschriebenen Methode. Statt Allopregnanolon wird 3-α-OH-CMA bzw. 3-β-OH-CMA verwendet.

Es kann gezeigt werden, dass bei einer Konzentration von 0,1 µM 3-β-OH-CMA die Bindung des radioaktiv markierten Muscimols (5 nM) um 31 % und bei einer Konzentration von 0,001 µM 3-α-OH-CMA um 12% erhöht wird.

Daraus kann auf einen stimmungsaufhellenden Effekt von 3-α-OH-CMA bzw. 3-β-OH-CMA geschlossen werden.

### Beispiele

### Beispiel 1

| **Zusammensetzung** | **Pro Tablette** |
|---|---|
| Östradiol | 1,00 mg |
| 3α-Hydroxychlormadinonacetat | 2,00 mg |
| 3β-Hydroxychlormadinonacetat | 2,00 mg |
| Povidon K30 | 3,000 mg |
| Lactose | 32,000 mg |
| Maisstärke | 0,500 mg |
| Hochdisperses Siliciumdioxid | 0,500 mg |

Östradiol und Povidone K 30 (PVP) wurden in 600 ml Ethanol gelöst. Die Gestagenkomponenten (Partikelgröße 90% <50µm), Lactose und Maisstärke wurden in einem Mischer/Granulierer (Diosna P25) 5 min gemischt und anschließend mit der ethanolischen Lösung enthaltend das Östradiol durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit hochdispersem Siliciumdioxid gemischt und auf einer Tablettenpresse mit 5 mm Stempeln zu Tabletten mit einem Gewicht von 50 mg gepreßt.

Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa · s, | 0,1351 kg |
| Polyethylenglykol 6000 | 0,0395 kg |
| Propylenglykol | 0,0054 kg |
| Gereinigtes Wasser | 1,6200 kg |

Diese überzogenen Tabletten wurden jeweils zu einem Kontrazeptivum mit 24 hormonhaltigen Tages-Einheiten und 4 entsprechend zusammengesetzten, hormonfreien, überzogenen Tabletten in einem Blister verpackt.

### Beispiel 2

| **Zusammensetzung** | **Pro Tablette** |
|---|---|
| Ethinylestradiol | 0,020 mg |
| Chlormadinonacetat | 1,00 mg |
| 3β-Hydroxychlormadinonacetat | 2,00 mg |
| 3α-Hydroxy-17α-acetoxy-5β-pregnan-20-on | 1,00 mg |
| Povidon K30 | 3,000 mg |
| Lactose | 31,980 mg |
| Maisstärke | 0,500 mg |
| Hochdisperses Siliciumdioxid | 0,500 mg |

Die Tabletten wurden, wie in Beispiel 1 angegeben, hergestellt und mit einem Lack der Zusammensetzung gemäß Beispiel 1 überzogen (Überzugsmasse 2 mg pro Tablette).

Die überzogenen Tabletten wurden zu einem Kontrazeptivum mit 24 hormonhaltigen Tages-Einheiten und 4 entsprechend zusammengesetzten, überzogenen, hormonfreien Tabletten in einem Blister verpackt.

## Patentansprüche

1. Eine Darreichungsform zur hormonalen Kontrazeption bestehend aus einer bestimmten Anzahl von hormonhaltigen Tages-Einheiten, die eine Hormonkombination bestehend aus wenigstens einem Östrogen auswählt aus der Gruppe bestehend aus Ethinylestradiol (I) und Östradiol (II) als Östrogenkomponente und aus wenigstens einem Metaboliten des Chlormadinonacetats ausgewählt aus der Gruppe umfassend 3α-Hydroxy-6-chlor-17α-acetoxy-4,6-pregnadien-20-on (3α-Hydroxy-chlormadinonacetat)_{;} 3β-Hydroxy-6-chlor-17α-acetoxy-4,6-pregnadien-20-on (3β-Hydroxychlormadinonacetat) gegebenenfalls gemischt mit Chlormadinonacetat und/oder 3α-Hydroxy-17α-acetoxy-5β-pregnan-20-on und/oder 3β-Hydroxy-17α-acetoxy-5β-pregnan-20-on als Gestagenkomponente aufweist zur ununterbrochenen, täglichen, oralen Verabreichung der hormonhaltigen Tages-Einheiten, gegebenenfalls in Kombination mit 7 bis 3 hormonfreien Tages-Einheiten.

2. Eine Darreichungsform nach Anspruch 1, **dadurch gekennzeichnet, dass** die Hormonkombination aus 5 bis 50 µg Ehinylestradiol und/oder 0,5 bis 4 mg Östradiol und 1 bis 10 mg der Gestagenkomponente und gegebenenfalls üblichen Hilfsstoffen besteht.

3. Eine Darreichungsform nach Anspruch 1 oder 2, **dadurch gekennzeichnet, dass** die Hormonkombination aus 5 bis 30 µg Ethinylestradiol und/oder 0,5 bis 2 mg Östradiol und 1 bis 10 mg der Gestagenkomponente und gegebenenfalls üblichen Hilfsstoffen besteht.

4. Eine Darreichungsform nach einem der Ansprüche 1 bis 3, **dadurch gekennzeichnet, dass** die Gestagenkomponente eine der folgenden Komponenten a) bis f)
a) 3α-Hydroxy-chlormadinonacetat oder
b) 3β-Hydroxy-chlormadinonacetat oder
c) eine Mischung von a) und b) in einem beliebigen Mischungsverhältnis oder
d) eine Mischung von a) und/oder b) mit bis zu 20 Gew.%, bezogen auf die Gesamtmischung von 3α-Hydroxy-17α-acetoxy-5β-pregnan-20-on und/oder 3β Hydroxy-17α-acetoxy-5β-pregnan-20-on, oder
e) eine Mischung von Chlormadinonacetat mit a) und/oder b) in Mischungsverhältnis von 10 bis 90 Gew.% Chlormadinonacetat und 90 bis 10 Gew.% von a) und/oder b), bezogen auf die Gesamtmischung, oder
f) eine Mischung von Chlormadinonacetat mit c) in einem Mischungsverhältnis von 10 bis 90 Gew.% Chlormadinonacetat und 90 bis 10 Gew.% von c), bezogen auf die Gesamtmischung und bis zu 20 Gew.%, bezogen auf die Gesamtmischung, 3α-Hydroxy-17α-acetoxy-5β-pregnan-20-on und/oder 3β-Hydroxy-17α-acetoxy-5β-pregnan-20-on, wobei die Gesamtmischung immer 100 Gew.% ergeben muss, ist.

5. Eine Darreichungsform gemäß einem der Ansprüche 1 bis 4, **dadurch gekennzeichnet, dass** eine Tages-Einheit aus einer Hormonkombination bestehend aus jeweils 15 µg, 20 µg oder 30 µg Ethinylestradiol und/oder 1 mg oder 2 mg Östradiol und jeweils 1, 2, 3, 4 oder 5 mg einer der Gestagenkomponenten a) bis f) und gegebenenfalls aus üblichen Hilfsstoffen besteht.

6. Eine Darreichungsform gemäß Anspruch 5, **dadurch gekennzeichnet, dass** die Hormonkombination aus jeweils 20 µg oder 30 µg Ethinylestradiol und/oder 1 mg Östradiol und ≥ 2 mg der Gestagenkomponenten a) bis f) besteht.

7. Eine Darreichungsform nach einem der Ansprüche 1 bis 6, **dadurch gekennzeichnet, dass** sie wenigstens 21 hormonhaltige Tages-Einheiten und gegebenenfalls 7 bis 3 hormonfreie Tages-Einheiten enthält.

8. Eine Darreichungsform nach Anspruch 7, **dadurch gekennzeichnet, dass** deren maximale Anzahl an hormonhaltigen Tages-Einheiten einer ununterbrochenen Verabreichung für mehrere Jahre, vorzugsweise für 2 Jahre, besonders bevorzugt für 1 Jahr, gegebenenfalls in Kombination mit 7 bis 3 hormonfreien Tages-Einheiten für eine ununterbrochene Verabreichung für 7 bis 3 Tagen entspricht.

9. Eine Darreichungsform nach Anspruch 8, **dadurch gekennzeichnet, dass** sie bis zu 730, vorzugsweise bis zu 365 hormonhaltigen Tages-Einheiten und gegebenenfalls 7 bis 3 hormonfreien Tages-Einheiten aufweist.

10. Eine Darreichungsform nach Anspruch 8, **dadurch gekennzeichnet, dass** sie 77 bis 193 hormonhaltige Tages-Einheiten gegebenenfalls in Kombination mit 7 bis 3 hormonfreien Tages-Einheiten aufweist.

11. Eine Darreichungsform nach Anspruch 8, **dadurch gekennzeichnet, dass** sie 42 bis 52 hormonhaltige Tages-Einheiten gegebenenfalls in Kombination mit 7 bis 3 hormonfreien Tages-Einheiten aufweist.

12. Eine Darreichungsform nach Anspruch 8, **dadurch gekennzeichnet, dass** sie 21 bis 25 hormonhaltige Tages-Einheiten gegebenenfalls in Kombination mit 7 bis 3 hormonfreien Tages-Einheiten, aufweist.

13. Eine Darreichungsform nach einem der Ansprüche 1 bis 12, **dadurch gekennzeichnet, dass** die hormonhaltigen Tages-Einheiten jeweils dieselbe Menge der Östrogenkomponente und jeweils dieselbe Menge der Gestagenkomponente aufweisen.

14. Eine Darreichungsform nach einem der Ansprüche 1 bis 13, **dadurch gekennzeichnet, dass** die Tages-Einheiten als Tabletten vorliegen.

15. Ein Kit enthaltend mindestens eine Darreichungsform zur hormonalen Kontrazeption nach einem der Ansprüche 1 bis 14.

16. Ein Kit nach Anspruch 14, **dadurch gekennzeichnet, dass** es mehrere Darreichungsformen nach einem der Ansprüche 1 bis 13 enthält.

17. Verwendung einer Hormonkombination aus Ethinylestradiol und/oder Östradiol als Östrogenkomponente und wenigstens einem Metaboliten des Chlormadinonacetats ausgewählt aus der Gruppe bestehend aus umfassend 3α-Hydroxy-6-chlor-17α-acetoxy-4,6-pregnadien-20-on (3α-Hydroxy-chlormadinonacetat), 3β-Hydroxy-6-chlor-17α-acetoxy-4,6-pregnadien-20-on (3β-Hydroxy-chlormadinonacetat), gegebenenfalls gemischt mit Chlormadinonacetat und/oder 3α-Hydroxy-17α-acetoxy-5β-pregnan-20-on und/oder 3β-Hydroxy-17α-acetoxy-5β-pregnan-20-on als Gestagenkomponente zur Herstellung eines oralen Kontrazeptivums zur oralen, monophasigen Hormonverabreichung für die Dauer von wenigstens 21 Tagen, gegebenenfalls in Kombination mit 7 bis 3 hormonfreien Tages-Einheiten.

## Claims

1. Administration form for hormonal contraception consisting of a particular number of hormone-containing daily units, comprising a hormone combination consisting of at least one oestrogen selected from the group consisting of ethynyl oestradiol (I) and oestradiol (II) as the oestrogen component, and at least one metabolite of chlormadinone acetate selected from the group comprising 3α-hydroxy-6-chloro-17α-acetoxy-4,6-pregnadien-20-one (3α-hydroxy-chlormadinone acetate), 3β-hydroxy-6-chloro-17α-acetoxy-4,6-pregnadien-20-one (3β-hydroxy-chlormadinone acetate) optionally mixed with chlormadinone acetate and/or 3α-hydroxy-17α-acetoxy-5β-pregnan-20-one and/or 3β-hydroxy-17α-acetoxy-5β-pregnan-20-one as the gestagen component for uninterrupted, daily, oral administration of the hormone-containing daily units, optionally in combination with 7 to 3 hormone-free daily units.

2. Administration form according to claim 1, **characterised in that** the hormone combination consists of between 5 and 50 µg ethynyl oestradiol and/or between 0.5 and 4 mg oestradiol and between 1 and 10 mg gestagen component and optionally conventional additives.

3. Administration form according to either claim 1 or claim 2, **characterised in that** the hormone combination consists of between 5 and 30 µg ethynyl oestradiol and/or between 0.5 and 2 mg oestradiol and between 1 and 10 mg gestagen component and optionally conventional additives.

4. Administration form according to any one of claims 1 to 3, **characterised in that** the gestagen component is one of the following components a) to f)
a) 3α-hydroxy-chlormadinone acetate or
b) 3β-hydroxy-chlormadinone acetate or
c) a mixture of a) and b) at any mixing ratio or
d) a mixture of a) and/or b) with up to 20 % by weight, based on the total mixture, of 3α-hydroxy-17α-acetoxy-5β-pregnan-20-one and/or 3β hydroxy-17α-acetoxy-5β-pregnan-20-one, or
e) a mixture of chlormadinone acetate with a) and/or b) at a mixing ratio of from 10 to 90 % by weight chlormadinone acetate and from 90 to 10 % by weight of a) and/or b), based on the total mixture, or
f) a mixture of chlormadinone acetate with c) at a mixing ratio of from 10 to 90 % by weight chlormadinone acetate and from 90 to 10 % by weight of c), based on the total mixture and up to 20 % by weight, based on the total mixture, of 3α-hydroxy-17α-acetoxy-5β-pregnan-20-one and/or 3β-hydroxy-17α-acetoxy-5β-pregnan-20-one, the total mixture always having to equal 100 % by weight.

5. Administration form according to any one of claims 1 to 4, **characterised in that** one daily unit consists of a hormone combination consisting in each case of 15 µg, 20 µg or 30 µg ethynyl oestradiol and/or 1 mg or 2 mg oestradiol and 1, 2, 3, 4 or 5 mg respectively of one of the gestagen components a) to f) and, optionally, conventional additives.

6. Administration form according to claim 5, **characterised in that** the hormone combination consists in each case of 20 µg or 30 µg ethynyl oestradiol and/or 1 mg oestradiol and ≥ 2 mg of the gestagen components a) to f).

7. Administration form according to any one of claims 1 to 6, **characterised in that** it contains at least 21 hormone-containing daily units and optionally 7 to 3 hormone-free daily units.

8. Administration form according to claim 7, **characterised in that** the maximum number of hormone-containing daily units corresponds to uninterrupted administration for several years, preferably for two years, particularly preferably for one year, optionally in combination with 7 to 3 hormone-free daily units for uninterrupted administration for 7 to 3 days.

9. Administration form according to claim 8, **characterised in that** it comprises up to 730, preferably up to 365 hormone-containing daily units and optionally 7 to 3 hormone-free daily units.

10. Administration form according to claim 8, **characterised in that** it comprises between 77 and 193 hormone-containing daily units, optionally in combination with 7 to 3 hormone-free daily units.

11. Administration form according to claim 8, **characterised in that** it comprises between 42 and 52 hormone-containing daily units, optionally in combination with 7 to 3 hormone-free daily units.

12. Administration form according to claim 8, **characterised in that** it comprises between 21 and 25 hormone-containing daily units, optionally in combination with 7 to 3 hormone-free daily units.

13. Administration form according to any one of claims 1 to 12, **characterised in that** the hormone-containing daily units each comprise the same amount of the oestrogen component and the same amount of the gestagen component.

14. Administration form according to any one of claims 1 to 13, **characterised in that** the daily units are in the form of tablets.

15. Kit containing at least one administration form for hormonal contraception according to any one of claims 1 to 14.

16. Kit according to claim 14, **characterised in that** it contains a plurality of administration forms according to any one of claims 1 to 13.

17. Use of a hormone combination of ethynyl oestradiol and/or oestradiol as the oestrogen component and at least one metabolite of chlormadinone acetate selected from the group comprising 3α-hydroxy-6-chloro-17α-acetoxy-4,6-pregnadien-20-one (3α-hydroxy-chlormadinone acetate), 3β-hydroxy-6-chloro-17α-acetoxy-4,6-pregnadien-20-one (3β-hydroxy-chlormadinone acetate), optionally mixed with chlormadinone acetate and/or 3α-hydroxy-17α-acetoxy-5β-pregnan-20-one and/or 3β-hydroxy-17α-acetoxy-5β-pregnan-20-one as the gestagen component for preparation of a monophase hormonal oral contraceptive to be administered for at least 21 days, optionally in combination with 7 to 3 hormone-free daily units.

## Revendications

1. Forme pharmaceutique pour la contraception hormonale, consistant en un certain nombre d'unités journalières contenant des hormones, qui comporte une association d'hormones consistant en au moins un oestrogène choisi dans le groupe constitué par l'éthinylestradiol (I) et l'oestradiol (II) en tant que composant oestrogène et en au moins un métabolite de l'acétate de chlormadinone choisi dans le groupe comprenant la 3α-hydroxy-6-chloro-17α-acétoxy-4,6-prégnadién-20-one (acétate de 3α-hydroxy-chlormadinone), la 3β-hydroxy-6-chloro-17α-acétoxy-4,6-prégnadién-20-one (acétate de 3β-hydroxy-chlormadinone) éventuellement en mélange avec de l'acétate de chlormadinone et/ou de la 3α-hydroxy-17α-acétoxy-5β-prégnan-20-one et/ou de la 3β-hydroxy-17α-acétoxy-5β-prégnan-20-one en tant que composant progestatif, pour l'administration orale, journalière, ininterrompue des unités journalières contenant des hormones, éventuellement en association avec 7 à 3 unités journalières sans hormones.

2. Forme pharmaceutique selon la revendication 1, **caractérisée en ce que** l'association d'hormones consiste en 5 à 50 µg d'éthinylestradiol et/ou 0,5 à 4 mg d'oestradiol et 1 à 10 mg du composant progestatif et éventuellement des adjuvants usuels.

3. Forme pharmaceutique selon la revendication 1 ou 2, **caractérisée en ce que** l'association d'hormones consiste en 5 à 30 µg d'éthinylestradiol et/ou 0,5 à 2 mg d'oestradiol et 1 à 10 mg du composant progestatif et éventuellement des adjuvants usuels.

4. Forme pharmaceutique selon l'une quelconque des revendications 1 à 3, **caractérisée en ce que** le composant progestatif est l'un des composants a) à f) suivants
a) acétate de 3α-hydroxy-chlormadinone ou
b) acétate de 3β-hydroxy-chlormadinone ou
c) un mélange de a) et b) en un rapport quelconque de mélange ou
d) un mélange de a) et/ou b) avec jusqu'à 20 % en poids, par rapport au mélange total, de 3α-hydroxy-17α-acétoxy-5β-prégnan-20-one et/ou 3β-hydroxy-17α-acétoxy-5β-prégnan-20-one, ou
e) un mélange d'acétate de chlormadinone avec a) et/ou b) en un rapport de mélange de 10 à 90 % en poids d'acétate de chlormadinone et 90 à 10 % en poids de a) et/ou b), par rapport au mélange total, ou
f) un mélange d'acétate de chlormadinone avec c) en un rapport de mélange de 10 à 90 % en poids d'acétate de chlormadinone et 90 à 10 % en poids de c), par rapport au mélange total, et jusqu'à 20 % en poids, par rapport au mélange total, de 3α-hydroxy-17α-acétoxy-5β-prégnan-20-one et/ou 3β-hydroxy-17α-acétoxy-5β-prégnan-20-one, le mélange total devant toujours faire 100 % en poids.

5. Forme pharmaceutique selon l'une quelconque des revendications 1 à 4, **caractérisée en ce qu'**une unité journalière consiste en une association d'hormones consistant en respectivement 15 µg, 20 µg ou 30 µg d'éthinylestradiol et/ou 1 mg ou 2 mg d'oestradiol et respectivement 1, 2, 3, 4 ou 5 mg d'un des composants progestatifs a) à f) et éventuellement des adjuvants usuels.

6. Forme pharmaceutique selon la revendication 5, **caractérisée en ce que** l'association d'hormones consiste en respectivement 20 µg ou 30 µg d'éthinylestradiol et/ou 1 mg d'oestradiol et ≥ 2 mg des composants progestatifs a) à f).

7. Forme pharmaceutique selon l'une quelconque des revendications 1 à 6, **caractérisée en ce qu'**elle contient au moins 21 unités journalières contenant des hormones et éventuellement 7 à 3 unités journalières sans hormones.

8. Forme pharmaceutique selon la revendication 7, **caractérisée en ce que** le nombre maximum d'unités journalières contenant des hormones correspond à une administration ininterrompue pendant plusieurs années, de préférence pendant 2 ans, de façon particulièrement préférée pendant 1 an, éventuellement en association avec 7 à 3 unités journalières sans hormones, pour une administration ininterrompue pendant 7 à 3 jours.

9. Forme pharmaceutique selon la revendication 8, **caractérisée en ce qu'**elle comporte jusqu'à 730, de préférence jusqu'à 365 unités journalières contenant des hormones et éventuellement 7 à 3 unités journalières sans hormones.

10. Forme pharmaceutique selon la revendication 8, **caractérisée en ce qu'**elle comporte 77 à 193 unités journalières contenant des hormones éventuellement en association avec 7 à 3 unités journalières sans hormones.

11. Forme pharmaceutique selon la revendication 8, **caractérisée en ce qu'**elle comporte 42 à 52 unités journalières contenant des hormones éventuellement en association avec 7 à 3 unités journalières sans hormones.

12. Forme pharmaceutique selon la revendication 8, **caractérisée en ce qu'**elle comporte 21 à 25 unités journalières contenant des hormones éventuellement en association avec 7 à 3 unités journalières sans hormones.

13. Forme pharmaceutique selon l'une quelconque des revendications 1 à 12, **caractérisée en ce que** les doses journalières contenant des hormones comportent chacune la même quantité du composant oestrogène et chacune la même quantité du composant progestatif.

14. Forme pharmaceutique selon l'une quelconque des revendications 1 à 13, **caractérisée en ce que** les doses journalières se trouvent sous forme de comprimés.

15. Nécessaire contenant au moins une forme pharmaceutique pour la contraception hormonale selon l'une quelconque des revendications 1 à 14.

16. Nécessaire selon la revendication 14, **caractérisé en ce qu'**il contient plusieurs formes pharmaceutiques selon l'une quelconque des revendications 1 à 13.

17. Utilisation d'une association d'hormones à base d'éthinylestradiol et/ou d'oestradiol en tant que composant oestrogène et d'au moins un métabolite de l'acétate de chlormadinone choisi dans le groupe comprenant la 3α-hydroxy-6-chloro-17α-acétoxy-4,6-prégnadién-20-one (acétate de 3α-hydroxy-chlormadinone), la 3β-hydroxy-6-chloro-17α-acétoxy-4,6-prégnadién-20-one (acétate de 3β-hydroxy-chlormadinone) éventuellement en mélange avec de l'acétate de chlormadinone et/ou de la 3α-hydroxy-17α-acétoxy-5β-prégnan-20-one et/ou de la 3β-hydroxy-17α-acétoxy-5β-prégnan-20-one en tant que composant progestatif, pour la fabrication d'un contraceptif oral pour l'administration orale monophasique d'hormones pendant la période d'au moins 21 jours, éventuellement en association avec 7 à 3 unités journalières sans hormones.
